# EUROPEAN PATENT APPLICATION

(11) **EP 2 944 256 A1**
(43) Date of publication of application: **18.11.2015**
(21) Application number: 14181440.0
(22) Date of filing: 19.08.2014
(51) Int. Cl.: A61B 6/00

(54) **Automatic x-ray exposure parameter control system with depth camera and method**

(30) Priority: 14.05.2014 TW 103116931
(71) Applicant: Swissray Asia Healthcare Co., Ltd., Taipei City 114 (TW)
(72) Inventor: Hu, Chi Min, 114 Taipei City (TW)
(74) Representative: Karakatsanis, Georgios

(57) **Abstract**

An automatic X-ray exposure parameter control system (200, 200a) and method for a medical equipment (100) are disclosed. The automatic X-ray exposure parameter control system (200, 200a) applies a first image pixel depth signal (d1) and a second image pixel depth signal (d2) of a target human body (4) acquired by a depth camera (6) to compute a thickness difference (dx). An X-ray energy computation and conversion unit (72) then searches a corresponding X-ray energy (x1, x2, x3,..., xn) corresponding to the thickness difference (dx) and supplies an X-ray energy output value (s1). Then, a processor unit (5) generates an X-ray energy control signal (s2) to control an X-ray energy generation unit (75) to select and output one of a plurality of exposure parameters (s21, s22, s23) to an X-ray source generator (1) to allow the X-ray source generator (1) to generate an X-ray beam (11) to the target human body (4).

## Description

### FIELD OF THE INVENTION

The present invention relates to an X-ray beam control system of a medical equipment, and in particular to an automatic X-ray exposure parameter control system with a depth camera for the medical equipment and a method.

### DESCRIPTION OF THE PRIOR ART

X-ray imaging systems have been commonly used in regular modern medical examinations. An X-ray imaging system generally comprises an X-ray source generator, a collimator, a detector, and a collimator control system. The detector is mounted at a location that is spaced from the X-ray source generator by a predetermined distance. The X-ray source generator projects an X-ray beam, which, after passing through a shielding plate and a filter arranged in the collimator, is projected to a target human body and is detector by the detector.

For the conventional facility that includes X-ray equipments, the X-ray source generator must be controlled with different exposure parameters according to different shapes and portions of human bodies, in order to generate a proper X-ray beam. In the known techniques, proper exposure parameters are determined according to the expertise of a radiographer or medical personnel. However, different results of the adjustment of the exposure parameter are often caused by various factors, including individual experiences, expertise, habits, and personal emotions.

It is now an issue to be attended by those involved in the business to resolve the above-discussed drawbacks.

### SUMMARY OF THE INVENTION

Thus, the overcome the above problems, an object of the present invention is to provide an automatic X-ray exposure parameter control system, which employs depth imaging techniques to detect a figure feature of a target human body and then to determine various exposure parameters of an X-ray beam generated by an X-ray source generator through conversion computation.

The technical solution adopted by the present invention to achieve the above object is that medical equipment is combined with an automatic X-ray exposure parameter control system, which applies a first image pixel depth signal and a second image pixel depth signal of a target human body acquired by a depth camera to compute a thickness difference. An X-ray energy computation and conversion unit then searches a corresponding exposure parameter value corresponding to the thickness difference and supplies an exposure parameter. Then, a processor unit generates an X-ray energy control signal to control an X-ray energy generation unit to select and output one of a plurality of exposure parameters to the X-ray source generator to allow the X-ray source generator to generate an X-ray beam to the target human body.

The exposure parameters comprise X-ray intensity parameter, X-ray flux parameter, and X-ray exposure time parameter for controlling the X-ray beam.

The automatic X-ray exposure parameter control system may comprise a data memory device, which stores at least one set of body thickness and corresponding X-ray energy data. The X-ray energy computation and conversion unit, upon receiving the thickness difference computed by the body thickness computation unit, conducts a search, in the data memory device, for at least one body thickness and corresponding X-ray energy data to which the thickness difference corresponds so as to generate an X-ray energy output value.

As to the efficacy, with the above-described technical solution adopted by the present invention, a radiographer or medical personnel, when opening an X-ray medical equipment, needs only to use the automatic X-ray exposure parameter control system of the present invention to readily obtain body features of a target human body and thus alleviates the problems resulting from insufficiency of experience and habits of the radiographer.

### BRIEF DESCRIPTION OF THE DRAWINGS

The present invention will be apparent to those skilled in the art by reading the following description of preferred embodiments of the present invention, with reference to the attached drawings, in which:
**Figure** 1 is a schematic view showing a circuit system according to a first embodiment of the present invention;
**Figure** 2 is an example illustrating values of body thickness and corresponding X-ray energy stored in a data memory device of Figure 1;
**Figure** 3 is a flow chart of the first embodiment of the present invention;
**Figure** 4 is a schematic view showing a circuit system according to a second embodiment of the present invention; and
**Figure** 5 is a flow chart of the second embodiment of the present invention.

### DETAILED DESCRIPTION OF THE PREFERRED EMBODIMENTS

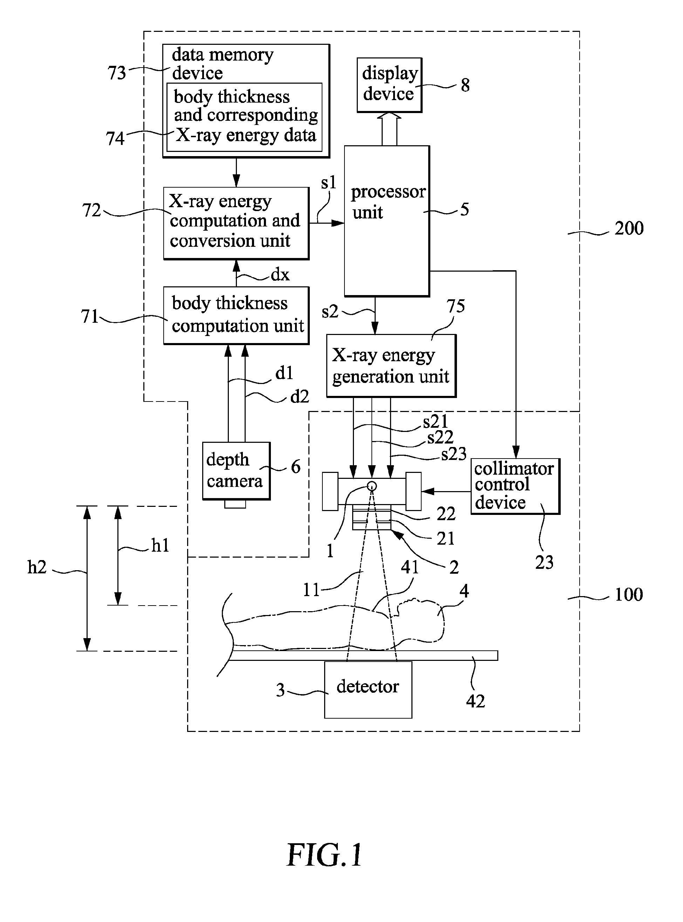

With reference to the drawings and in particular to Figure 1, which shows a first embodiment of the present invention, medical equipment 100 is arranged to include an X-ray source generator 1, a collimator 2, and a detector 3. The X-ray source generator 1 is combined with the collimator 2 and the detector 3 is arranged at a location that is spaced from the X-ray source generator 1 by a predetermined distance. The X-ray source generator 1 projects an X-ray beam 11, which after passing through a shielding plate 21 and a filter 22 arranged in the collimator 2, is projected to a target human body 4 and detected by the detector 3.

The collimator 2 is connected to a collimator control device 23, so that the collimator control device 23 controls the shielding plate 21 arranged in the collimator 2 to adjust a selected human body portion 41 of the target human body 4.

In the present invention, an automatic exposure parameter control system 200 is provided and combined with the medical equipment 100, so that the automatic exposure parameter control system 200 controls various exposure parameters of the X-ray source generator 1 of the medical equipment 100.

The automatic exposure parameter control system 200 according to the present invention comprises a processor unit 5, a depth camera 6, a body thickness computation unit 71, an X-ray energy computation and conversion unit 72, a data memory device 73, an X-ray energy generation unit 75, and a display device 8.

The depth camera 6 is set at a location corresponding to the target human body 4 in order to acquire a first thickness d1 of the depth camera 6 with respect to the target human body 4 and transmit a first image pixel depth signal h1 and also to acquire a second thickness d2 of the depth camera 6 with respect to a reference surface 42 and transmit a second image pixel depth signal h2.

Figure 1 shows that the first thickness d1 is defined as a thickness of the depth camera 6 with respect to the target human body 4 and the second thickness d2 is defined as a thickness of the depth camera 6 with respect to a table (which is the reference surface 42) on which the target human body 4 lies.

The body thickness computation unit 71 is connected to the depth camera 6 to receive the first image pixel depth signal h1 and the second image pixel depth signal h2 acquired by the depth camera 6 and computes, based on a difference between the first image pixel depth signal h1 and the second image pixel depth signal h2, a thickness difference dx (namely d2-d1=dx). Through the computation performed by the body thickness computation unit 71, body thickness data of the target human body 4 can be determined.

The X-ray energy computation and conversion unit 72 is connected to the processor unit 5 and the body thickness computation unit 71. The thickness difference dx obtained through the computation of the body thickness computation unit 71 is transmitted to the X-ray energy computation and conversion unit 72.

The data memory device 73 is also connected to the X-ray energy computation and conversion unit 72. The data memory device 73 stores therein a plurality of sets of body thickness and corresponding X-ray energy data 74.

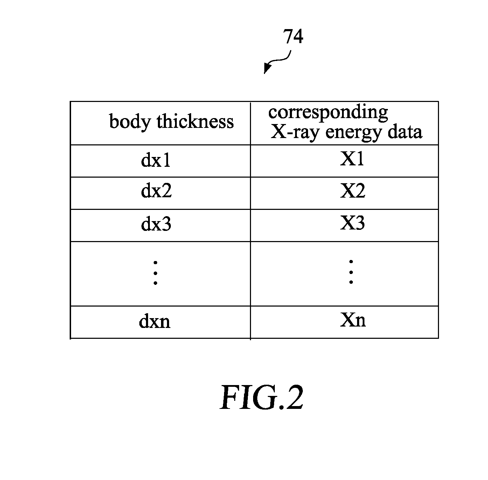

Figure 2 illustrates an example of the body thickness and corresponding X-ray energy data stored in the data memory device 73 and it is shown that the body thickness and corresponding X-ray energy data comprises a plurality of body thicknesses dx1, dx2, dx3, ..., dxn and X-ray energies x1, x2, x3, ..., xn respectively corresponding to the body thicknesses.

The X-ray energy computation and conversion unit 72, when receiving the thickness difference dx computed by the body thickness computation unit 71, conducts a search, based the thickness difference dx, for those of the thickness differences dx1, dx2, dx3, ..., dxn and X-ray energies x1, x2, x3, ..., xn of the data memory device 73 that correspond to the thickness difference dx and then transmits an X-ray energy output value s1 to the processor unit 5.

The processor unit 5, upon receiving the X-ray energy output value s1 transmitted from the X-ray energy computation and conversion unit 72, generates an X-ray energy control signal s2 to the X-ray energy generation unit 75, so that the X-ray energy generation unit 75 selects and transmits, according to the X-ray energy control signal s2, one of exposure parameters s21, s22, s23 to the X-ray source generator 1 to allow the X-ray source generator 1 to generate, based on the exposure parameter, an X-ray beam 11 aiming at a selected human body portion 41 of the target human body 4.

In an embodiment of the present invention, the exposure parameters s21, s22, s23 includes one of an X-ray intensity parameter, an X-ray flux parameter, and an X-ray exposure time parameter or a combination thereof to control the X-ray beam.

With the control operation conducted above, the X-ray source generator can be controlled according to different exposure parameters to generate an X-ray beam with optimum exposure parameters to be projected to the target human body.

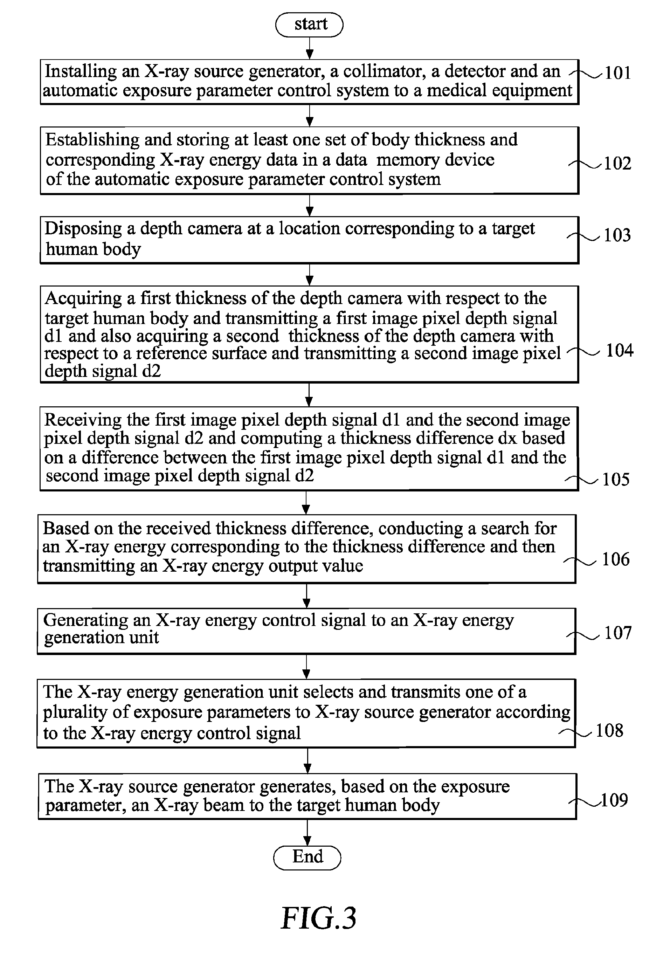

Figure 3 illustrates a flow chart of the first embodiment of the present invention. An operational flow of the present invention will be described with additional reference to Figures 1 and 2. Firstly, medical equipment 100 is arranged to comprises an X-ray source generator 1, a collimator 2, a detector 3 and an automatic exposure parameter control system 200 according to the present invention (Step 101) and at least one set of body thickness and corresponding X-ray energy data (Step 102) is established and stored in a data memory device 73 of the automatic exposure parameter control system 200 according to the present invention and a depth camera 6 is disposed at a location corresponding to a target human body 4 (Step 103).

With the target human body 4 being set at a proper location corresponding to the X-ray source generator 1, the depth camera 6 is operated to acquire a first thickness d1 of the depth camera 6 with respect to the target human body 4 and to transmit a first image pixel depth signal h1 and also to acquire a second thickness d2 of the depth camera 6 with respect to a reference surface 42 and to transmit a second image pixel depth signal h2 (Step 104).

A body thickness computation unit 71 is operated to receive the first image pixel depth signal h1 and the second image pixel depth signal h2 and to compute a thickness difference dx based on a difference between the first image pixel depth signal h1 and the second image pixel depth signal h2 (namely d2-d1=dx) (Step 105).

An X-ray energy computation and conversion unit 72, upon receiving the thickness difference dx, conducts a search, based the thickness difference dx, for those of thickness differences dx1, dx2, dx3, ..., dxn and X-ray energies x1, x2, x3, ..., xn of the data memory device 73 that corresponding to the thickness difference dx and then transmits an X-ray energy output value s1 to a processor unit 5 (Step 106).

The processor unit 5, upon receiving the X-ray energy output value s1 transmitted from the X-ray energy computation and conversion unit 72, generates an X-ray energy control signal s2 to an X-ray energy generation unit 75 (Step 107), so that the X-ray energy generation unit 75 selects, according to the X-ray energy control signal s2, and transmit one of a plurality of exposure parameters s21, s22, s23 to the X-ray source generator 1 (Step 108) to allow the X-ray source generator 1 to generate, based on the exposure parameter, an X-ray beam 11 aiming at a selected human body portion 41 of the target human body 4 (Step 109).

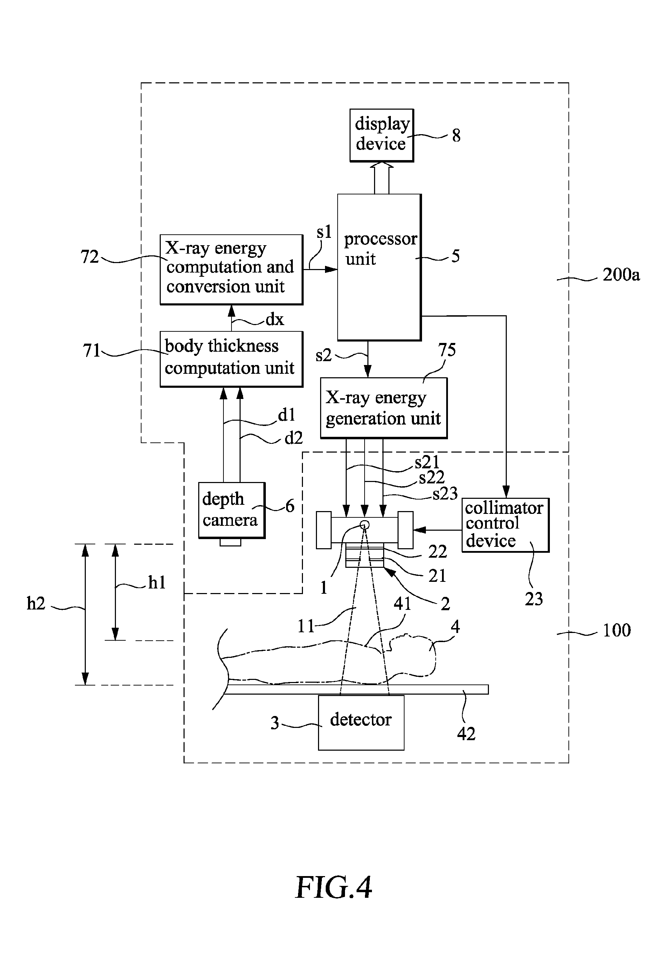

Referring to Figure 4, a schematic view is given to show a circuit system according to a second embodiment of the present invention. The instant embodiment comprises constituent components that are substantially identical to those of the first embodiment and identical components are designated with similar reference numerals for consistency. In the instant embodiment, the medical equipment 100 is similarly arranged to include an X-ray source generator 1, a collimator 2, and a detector 3. An automatic exposure parameter control system 200a comprises a processor unit 5, a depth camera 6, a body thickness computation unit 71, an X-ray energy computation and conversion unit 72, an X-ray energy generation unit 75, and a display device 8.

In the instant embodiment, the X-ray energy computation and conversion unit 72, upon receiving a thickness difference dx computed by the body thickness computation unit 71, computes and generates, according to the thickness difference dx, an X-ray energy output value s1 that corresponds to the thickness difference dx and is transmitted to the processor unit 5.

The processor unit 5, upon receiving the X-ray energy output value s1 transmitted from the X-ray energy computation and conversion unit 72, generates an X-ray energy control signal s2 that is transmitted to the X-ray energy generation unit 75, so that the X-ray energy generation unit 75 selects and transmits, according to the X-ray energy control signal s2, one of exposure parameters s21, s22, s23 to the X-ray source generator 1 to allow the X-ray source generator 1 to generate, based on the exposure parameter, an X-ray beam 11 aiming at a selected human body portion 41 of a target human body 4.

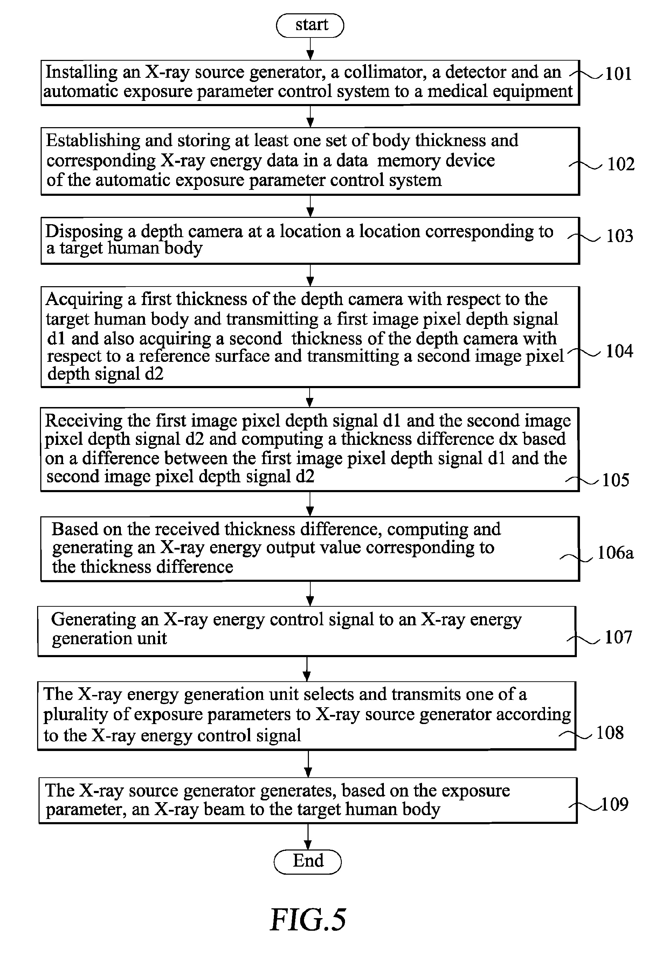

Figure 5 is a flow chart of the second embodiment of the present invention, which comprises steps that are substantially identical to those of the first embodiment and identical steps are designated with the same reference numerals for consistency, wherein a difference resides in that in Step 106a, the X-ray energy computation and conversion unit 72, upon receiving the thickness difference dx, computes and generates, according to the thickness difference dx, an X-ray energy output value s1 that corresponds to the thickness difference dx and is transmitted to the processor unit 5.

Although the present invention has been described with reference to the preferred embodiments thereof, it is apparent to those skilled in the art that a variety of modifications and changes may be made without departing from the scope of the present invention which is intended to be defined by the appended claims.

## Claims

1. An automatic X-ray exposure parameter control system (200, 200a) for a medical equipment (100), in which the medical equipment (100) includes an X-ray source generator (1), a collimator (2), a collimator control device (23), and a detector (3), wherein the X-ray source generator (1) projects an X-ray beam (11) to a target human body (4) through the collimator (2) and the detector (3) detects the X-ray beam (11), **characterized in that** the automatic X-ray exposure parameter control system (200, 200a) comprises:
a processor unit (5);
a depth camera (6), which is connected to the processor unit (5) and is set at a location corresponding to the target human body (4) in order to acquire a first image pixel depth signal (d1) of the depth camera (6) with respect to the target human body (4) and a second image pixel depth signal (d2) of the depth camera (6) with respect to a reference surface (42);
a body thickness computation unit (71), which is connected to the depth camera (6), the body thickness computation unit (71) receiving the first image pixel depth signal (d1) and the second image pixel depth signal (d2) acquired by the depth camera (6) and computing a thickness difference (dx) according to a difference between the first image pixel depth signal (d1) and the second image pixel depth signal (d2);
an X-ray energy computation and conversion unit (72), which is connected to the processor unit (5) and the body thickness computation unit (71), the X-ray energy computation and conversion unit (72) receiving the thickness difference (dx) computed by the body thickness computation unit (71) and transmitting an X-ray energy output value (s1) according to the thickness difference (dx) to the processor unit (5); and
an X-ray energy generation unit (75), which is connected to the processor unit (5) and the X-ray source generator (1), the X-ray energy generation unit (75) generating a plurality of different exposure parameters (s21, s22,
s23) supplied to the X-ray source generator (1);
wherein the processor unit (5), upon receiving the X-ray energy output value (s1) generated by the X-ray energy computation and conversion unit (72), generates and transmits an X-ray energy control signal (s2) to the X-ray energy generation unit (75), so that the X-ray energy generation unit (75) selects and outputs, according to the X-ray energy control signal (s2), one of the exposure parameters (s21, s22, s23) to the X-ray source generator (1) to allow the X-ray source generator (1) to generate an X-ray beam (11) according to the exposure parameter (s21, s22, s23).

2. The automatic X-ray exposure parameter control system as claimed in Claim 1, wherein the exposure parameters (s21, s22, s23) comprise X-ray intensity parameter, X-ray flux parameter, and X-ray exposure time parameter for controlling the X-ray beam (11).

3. The automatic X-ray exposure parameter control system as claimed in Claim **1** further comprising a data memory device (73), which is connected to the processor unit (5) and stores therein at least one set of body thickness and corresponding X-ray energy data (74), the X-ray energy computation and conversion unit (72) receiving the thickness difference (dx) computed by the body thickness computation unit (71) and conducting a search, in the data memory device (73), for at least one set of body thickness and corresponding X-ray energy data (74) that corresponds to the thickness difference (dx) in order to generate the X-ray energy output value (s1).

4. An automatic X-ray exposure parameter control method for a medical equipment (100), in which the medical equipment (100) includes an X-ray source generator (1), a collimator (2), and a detector (3), **characterized in that** the method comprises the following steps:
(a) establishing and storing at least one set of body thickness and corresponding X-ray energy data (74);
(b) setting a depth camera (6) to correspond to a target human body (4) in order to acquire a first thickness (h1) of the depth camera (6) with respect to the target human body (4) and transmit a first image pixel depth signal (d1) and to acquire a second thickness (h2) of the depth camera (6) with respect to a reference surface (42) and transmits a second image pixel depth signal (d2);
(c) computing a thickness difference (dx) according to a difference between the first image pixel depth signal (d1) and the second image pixel depth signal (d2);
(d) conducting a search, based on the thickness difference (dx), for an X-ray energy (x1, x2, x3,..., xn) corresponding to the thickness difference (dx) and transmitting an X-ray energy output value (s1);
(e) generating an X-ray energy control signal (s2) according to the X-ray energy output value (s1);
(f) selecting and outputting one of a plurality of exposure parameters (s21, s22, s23) to the X-ray source generator (1) according to the X-ray energy control signal (s2); and
(g) operating the X-ray source generator (1) to generate an X-ray beam (11) according to the exposure parameter (s21, s22, s23) to be projected to the target human body (4).

5. The automatic X-ray exposure parameter control method as claimed in Claim **4,** wherein the reference surface (42) is provided by setting a table on which the target human body (4) lies as the reference surface (42).

6. The automatic X-ray exposure parameter control method as claimed in Claim **4,** wherein the exposure parameters (s21, s22, s23) comprise X-ray intensity parameter, X-ray flux parameter, and X-ray exposure time parameter for controlling the X-ray beam (11).

7. An automatic X-ray exposure parameter control method for a medical equipment (100), in which the medical equipment (100) includes an X-ray source generator (1), a collimator (2), and a detector (3), **characterized in that** the method comprises the following steps:
(a) setting a depth camera (6) to correspond to a target human body (4) in order to acquire a first thickness (h1) of the depth camera (6) with respect to the target human body (4) and transmit a first image pixel depth signal (d1) and to acquire a second thickness (h2) of the depth camera (6) with respect to a reference surface (42) and transmits a second image pixel depth signal (d2);
(b) computing a thickness difference (dx) according to a difference between the first image pixel depth signal (d1) and the second image pixel depth signal (d2);
(c) generating an X-ray energy output value (s1) according to the thickness difference (dx);
(d) generating an X-ray energy control signal (s2) according to the X-ray energy output value (s1);
(e) selecting and outputting one of a plurality of exposure parameters (s21, s22, s23) to the X-ray source generator (1) according to the X-ray energy control signal (s1); and
(f) operating the X-ray source generator (1) to generate an X-ray beam (11) according to the exposure parameter (s21, s22, s23) to be projected to the target human body (4).

8. The automatic X-ray exposure parameter control method as claimed in Claim **7,** wherein the reference surface (42) is provided by setting a table on which the target human body (4) lies as the reference surface (42).

9. The automatic X-ray exposure parameter control method as claimed in Claim **7,** wherein the exposure parameters (s21, s22, s23) comprise X-ray intensity parameter, X-ray flux parameter, and X-ray exposure time parameter for controlling the X-ray beam (11).
